(19) Europäisches Patentamt
European Patent Office
Office européen des brevets

(11) **EP 2 988 661 B1**

(12) **EUROPÄISCHE PATENTSCHRIFT**

(45) Veröffentlichungstag und Bekanntmachung des Hinweises auf die Patenterteilung:
**14.06.2017 Patentblatt 2017/24**

(21) Anmeldenummer: **14719281.9**

(22) Anmeldetag: **17.04.2014**

(51) Int Cl.:
*A61B 5/0215* (2006.01)   *A61M 1/36* (2006.01)

(86) Internationale Anmeldenummer:
**PCT/EP2014/057952**

(87) Internationale Veröffentlichungsnummer:
**WO 2014/173828 (30.10.2014 Gazette 2014/44)**

(54) **STEUEREINHEIT UND VERFAHREN ZUR BESTIMMUNG EINES DRUCKS IN EINEM BLUTGEFÄSS, INSBESONDERE IN EINER ARTERIOVENÖSEN FISTEL**

CONTROL UNIT AND METHOD FOR DETERMINING A PRESSURE IN A BLOOD VESSEL, ESPECIALLY IN AN ARTERIOVENOUS FISTULA

UNITÉ DE COMMANDE ET PROCÉDÉ DE DÉTERMINATION D'UNE PRESSION DANS UN VAISSEAU SANGUIN, EN PARTICULIER DANS UNE FISTULE ARTÉRIO-VEINEUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.04.2013 DE 102013007044**

(43) Veröffentlichungstag der Anmeldung:
**02.03.2016 Patentblatt 2016/09**

(73) Patentinhaber: **Fresenius Medical Care Deutschland GmbH**
**61352 Bad Homburg (DE)**

(72) Erfinder: **KOPPERSCHMIDT, Pascal**
**97456 Dittelbrunn (DE)**

(74) Vertreter: **Dreyhsig, Jörg**
**Fresenius Medical Care AG**
**& Co. KGaA**
**Global Patents & IP**
**Frankfurter Straße 6-8**
**66606 St. Wendel (DE)**

(56) Entgegenhaltungen:
**EP-A1- 2 009 415       DE-A1- 4 024 434**
**DE-A1-102008 059 379    DE-C1- 19 917 197**

EP 2 988 661 B1

**Beschreibung**

Die Erfindung betrifft eine Steuereinheit zur Bestimmung eines Drucks in einem Blutgefäß, insbesondere in einer arteriovenösen Fistel, ein Verfahren zur Bestimmung eines Drucks in einem Blutgefäß und eine Blutbehandlungsvorrichtung.

Bei Verfahren der chronischen Blutreinigungstherapie wird Blut über einen extrakorporalen Blutkreislauf geleitet. Bei der Hämodialyse (HD) wird das Blut durch einen Dialysator gereinigt, der eine im extrakorporalen Blutkreislauf befindliche Blutkammer und eine Dialysierflüssigkeitskammer aufweist, die durch eine semipermeable Membran voneinander getrennt sind. Die Dialysierflüssigkeitskammer wird während einer Hämodialysebehandlung von Dialysierflüssigkeit durchflossen, wobei bestimmte Substanzen aufgrund der Diffusion zwischen dem Blut und der Dialysierflüssigkeit durch die Membran transportiert und mit der Dialysierflüssigkeit über einen Dialysierflüssigkeitskreislauf entfernt werden. Bei der Hämofiltration (HF) werden bestimmte Substanzen aufgrund von Konvektion durch eine Filtermembran aus dem Blut gefiltert. Die Hämodiafiltration (HDF) stellt eine Kombination aus beiden Verfahren dar.

Dem Patienten bei den Blutreinigungsverfahren entzogene Flüssigkeit lässt sich durch eine Substitutionsflüssigkeit ersetzen, die dem extrakorporalen Blutkreislauf während der Blutbehandlung zugeführt wird.

Bei der Blutbehandlung kommen Schlauchleitungssysteme zum Einsatz, die in die Blutbehandlungsvorrichtung, wie etwa ein HDF-Gerät, eingelegt werden.

Extrakorporale Blutbehandlungsvorrichtungen enthalten mehrere Pumpen, die das Blut des Patienten und die Substitutionsflüssigkeit in den Schlauchleitungen der Schlauchleitungssysteme fördern. Es finden vor allem peristaltische Pumpen Verwendung, bei denen sich mindestens eine Eng- oder Verschlussstelle längs des als Pumpenraum dienenden elastischen Schlauchs bewegt. Bei der gebräuchlichsten Bauart der peristaltischen Schlauchpumpen wird der elastische Schlauch an der Eng- oder Verschlussstelle vollständig verschlossen. Diese Pumpen werden daher auch als okkludierende Schlauchpumpen bezeichnet. Die gebräuchlichste okkludierende Schlauchpumpe ist eine Rollenpumpe, in die ein Abschnitt einer Schlauchleitung des Schlauchleitungssystems eingelegt wird.
Im Dialysierflüssigkeitskreislauf befindet sich eine Dialysierflüssigkeitspumpe zur Förderung der Dialysierflüssigkeit. Eine Ultrafiltrationspumpe erzeugt den nötigen Unterdruck in der Dialysierflüssigkeitskammer des Dialysators, damit dem Patienten Flüssigkeit zur Erhaltung des Flüssigkeitshaushalts entfernt werden kann, die nicht durch Substitutionsflüssigkeit ersetzt wird.
Als Zugang zum Blutgefäßsystem des Patienten wird häufig operativ eine arteriovenöse Fistel angelegt, die im Allgemeinen mit einer arteriellen und einer venösen Kanüle punktiert wird. Alternativ ist der Einsatz eines Gefäßimplantats, wie etwa eines sogenannten Goretex-

Grafts oder eines sogenannnen PTFE-Shunts, möglich. Im Folgenden wird unter einer "Fistel" jegliche Art der Verbindung zwischen einer Vene und einer Arterie eines Patienten verstanden.

Für die Funktionsfähigkeit der Fistel ist deren Perfusion von großer Bedeutung. Sinkt der Fistelfluss unter einen kritischen Wert, dann steigt das Risiko einer Fistelthrombose, verbunden mit der Gefahr, dass der für die Blutreinigungstherapie essentielle Gefäßzugang verloren wird. Ein sinkender Fistelfluss kann durch eine sich entwickelnde Zu- oder Abflussstenose der Fistel, eine Kalzifizierung der Fistel, eine Füllung der Fistel und weitere ähnliche Ursachen begründet sein, die im Folgenden unter dem Begriff Fistelstenose zusammengefasst werden.

Um die negativen Folgen einer Fistelstenose zu vermeiden, ist es erwünscht, eine sich entwickelnde Fistelstenose möglichst weit im Vorfeld zu entdecken oder zumindest bevor ein kritischer Stenosengrad erreicht ist.

Zu diesem Zwecke wurden verschiedene Verfahren vorgeschlagen. Eine Gruppe von Verfahren beschäftigt sich mit der Messung des Blutflusses. In der DE 19917197 C1 wird ein Verfahren und eine Vorrichtung zur Ermittlung des Blutflusses $Q_F$ in einem Gefäßzugang F während einer extrakorporalen Blutbehandlung beschrieben. Die Bestimmung im Gefäßzugang beruht darauf, dass der Druck in einem arteriellen und/oder venösen Zweig des extrakorporalen Kreislaufs bei offenem und unterbrochenem Gefäßzugang gemessen wird, während der extrakorporale Blutfluss $Q_B$ verändert wird. Aus den gemessenen Werten des arteriellen und/oder venösen Drucks bei offenem und unterbrochenem Gefäßzugang wird dann der Fistelfluss $Q_F$ bestimmt.

Die DE 102008061122 A1 betrifft ein Verfahren zum Ermitteln und/oder Überwachen von kardiovaskulären Größen, den körperlichen Zustand eines Patient betreffend und eine Vorrichtung zum Messen einer Amplitude eines Herzdrucksignals. Dabei kann der körperliche Zustand eine Fisteldurchgängigkeit sein.

Die DE102008059379A1 beschreibt eine Vorrichtung und ein Verfahren zur invasiven Blutdruckmessung im Gefäßzugang bei kontinuierlichen Blutflüssen in einem Therapiegerät bei extracorporalen Entgiftungsverfahren. Dabei wird eine Messung der Absolutwerte des Blutdrucks, die Steilheit des Druckanstiegs der mittleren arteriellen Blutdruckwerte bis zum Erreichen einer Plateauphase und der Anstieg der absolut gemessenen Blutdruckwerte, sowie die Änderungen der Pulsvolumenkurven in dem Gefäßzugang bei Unterbrechung des Blutstroms durchgeführt, wobei eine elektronische Auswertung die Änderung der gemessenen Werte nach Umschaltung in den Bypass-Modus bis auf eine Plateauphase berücksichtigt.

Aufgabe der vorliegenden Erfindung ist, ein einfach durchzuführendes Verfahren zur genauen Bestimmung des Fisteldrucks anzugeben, sowie eine Vorrichtung hierzu anzugeben. Es ist ferner eine Aufgabe der vorliegenden Erfindung, eine Vorrichtung zur Vorhersage einer beginnenden Fistelstenose und ein entsprechendes Verfahren anzugeben.

Die Aufgabe wird durch eine Steuereinheit mit den Merkmalen von Anspruch 1 gelöst. Vorteilhafte Ausführungsformen sind in den Unteransprüchen 2 bis 12 gekennzeichnet. Eine erfindungsgemäße Steuereinheit zur Bestimmung eines Drucks in einem Blutgefäß, das in Fluidverbindung mit zumindest einem Abschnitt eines Blutleitungssystems, insbesondere eines extrakorporalen Blutkreislaufs, steht, wobei dem Blutleitungssystem zumindest eine Druckerzeugungseinrichtung zugeordnet ist, die geeignet ist, auf den Abschnitt einzuwirken, ist konfiguriert, die folgenden Schritte durchzuführen:

a) Sicherstellen, dass keine der zumindest einen Druckerzeugungseinrichtung auf den Abschnitt einwirkt,
b) Unterbrechen der Fluidverbindung des Abschnitts mit dem Blutgefäß durch Ansteuern eines Unterbrechungsmittels,
c) Einstellen des Drucks in dem Abschnitt auf einen vorbestimmten Sollwert, insbesondere auf den Umgebungsdruck, mit Hilfe eines Drucksensors im Abschnitt,
d) Wiederherstellen der Fluidverbindung des Abschnitts mit dem Blutgefäß durch Ansteuern des Unterbrechungsmittels und
e) Messen eines sich einstellenden Drucks im Abschnitt mit Hilfe des Drucksensors.

Der Druck im Blutgefäß kann im Rahmen der Erfindung aufgrund eines wellenförmigen Druckverlaufs im Blutgefäßsystem und somit bei Fluidverbindung auch im Blutleitungssystem auf verschiedene Arten definiert sein. Bevorzugt wird unter dem "Druck im Blutgefäß" und dem "Druck im Abschnitt" jeweils der Mittelwert des aufgrund des Herzschlags des Patienten wellenförmig verlaufenden Drucks verstanden.

Entsprechend der Verwendung der ansonsten nicht mehr gebräuchlichen Druckeinheit "mmHg" zur Angabe von Blutdruck in der Medizin, der als Differenz des Drucks in einem Blutgefäß gegenüber dem Umgebungsdruck definiert ist, wird im Rahmen der Erfindung zur Wiedergabe von Druckwerten im Blutleitungssystem ebenfalls der Druck als Relativdruck gegenüber dem Umgebungsdruck mit der Einheit "mmHg" verwendet. Ein Druck von 0 mmHg im Blutleitungssystem entspricht gemäß dieser Definition dem Umgebungsdruck.

Vorzugsweise wird mit der Steuereinheit der Druck in einer arteriovenösen Fistel bestimmt bzw. ist diese dazu konfiguriert. In diesem Fall wird der Druck im Blutgefäß als Fistelinnendruck, kurz Fisteldruck, bezeichnet. Entsprechend den Ausführungen oben ist der Fisteldruck von der erfindungsgemäßen Steuereinheit als ein Mittelwert aus maximalem Fisteldruck und minimalem Fisteldruck bestimmbar.

Die Erfindung hat den Vorteil, dass die Messung des Drucks in einem Abschnitt eines Blutleitungssystems, der gegenüber störenden Einflüssen isoliert ist, von einem vorbestimmten Sollwert ausgehend ausgeführt wird. Dadurch werden Fehlerquellen, die die Genauigkeit des gemessenen Drucks beeinflussen, ausgeschlossen oder zumindest minimiert. Ausgeschlossene bzw. minimierte Fehlerquellen können beispielsweise sein, dass sich ausgehend von verschieden hohen Ausgangsdrücken bei verschiedenen Messungen oder von Ausgangsdrücken mit unterschiedlichen Vorzeichen im Abschnitt jeweils andere tatsächlich gemessene Drücke einstellen. Auch stellen sich bei derartigen verschiedenen Ausgangsdrücken Drücke mit verschiedenen Geschwindigkeiten ein. Dies hätte zur Folge, dass sich bei einer Messung nach einer vorbestimmten Zeit trotz gleicher Enddrücke andere tatsächlich gemessene Drücke ergeben. Mittels der erfindungsgemäßen Steuereinheit kann ausgehend vom vorteilhafterweise stets selben vorbestimmten Druck im Abschnitt der Druck als sich bis zum Abschnitt übertragender Druck im Blutgefäß bestimmt werden, der lediglich durch einen hydrostatischen Druck überlagert ist. In der bevorzugten Ausführungsform, bei der die Steuereinheit konfiguriert ist, den Druck im Abschnitt in Schritt c) auf Umgebungsdruck einzustellen, lässt sich der sich einstellende Druck in Schritt e) mit einer besonders hohen Genauigkeit bestimmen.

Der Schritt des "Sicherstellens", dass keine der zumindest einen Druckerzeugungseinrichtung auf den Abschnitt einwirkt, umfasst nach Art eines Steuerschritts einen ersten Fall, dass bereits ein Nicht-Einwirken vorliegt und dies von der Steuereinheit lediglich festgestellt werden muss, und einen zweiten Fall, dass das Nicht-Einwirken von der Steuereinheit erst noch herbeigeführt werden muss.

Im ersten Fall wirkt diejenige zumindest eine Druckerzeugungseinrichtung, die geeignet sind, auf den Abschnitt einzuwirken, bereits nicht auf den Abschnitt ein. Dies kann die Steuereinheit gemäß einer ersten Variante dadurch feststellen, dass sie ein Signal von der Druckerzeugungseinrichtung erhält, dass diese bereits angehalten wurde. Auch kann die Steuereinheit konfiguriert sein, einen Status des Nicht-Einwirkens dann als gegeben anzunehmen, wenn ein mit der Steuereinheit verbundener Drucksensor, der geeignet ist, den Druck im Abschnitt zu messen, der Steuereinheit die Information liefert, dass keine Druckveränderung im Abschnitt stattfindet. Weiterhin kann die Steuereinheit konfiguriert sein, einen Status des Nicht-Einwirkens der Druckerzeugungseinrichtungen auf den Abschnitt anzunehmen, wenn zumindest eine Unterbrechungseinrichtung den Abschnitt gegenüber zumindest einer Druckerzeugungseinrichtung absperrt.

Beim zweiten Fall wirkt die zumindest eine Druckerzeugungseinrichtung noch auf den Abschnitt ein. Für diesen Fall kann die Steuereinheit konfiguriert sein, dies durch Ansteuern von entsprechenden Mitteln, wie etwa der zumindest einen Druckerzeugungseinrichtung, einem Drucksensor und/oder zumindest einem Unterbrechungsmittel, festzustellen. Die Steuereinheit stellt in diesem Fall dadurch sicher, dass die zumindest eine Druckerzeugungseinrichtung nicht auf den Abschnitt einwirkt, dass sie ein Mittel ansteuert, das die Einwirkung beendet. Dieses anzusteuernde Mittel kann wiederum die zumindest eine Druckerzeugungseinrichtung, die noch auf den Abschnitt einwirkt, und/oder zumindest eine Unterbrechungseinrichtung, die geeignet ist, den Abschnitt von der zumindest einen Druckerzeugungseinrichtung abzusperren, sein. Die Druckerzeugungseinrichtung kann beispielsweise abgeschaltet, gestoppt oder in einen Leerlaufmodus versetzt werden, damit sie nicht mehr auf den Abschnitt einwirkt. Unter einem Einwirken einer Druckerzeugungseinrichtung auf den Abschnitt ist im Rahmen der Erfindung jede Art von Druckveränderung zu verstehen. Ein Abschalten, Stoppen oder Leerlaufbetrieb der Druckerzeugungseinrichtung ist besonders energiesparend, verhindert den Aufbau starker Druckdifferenzen im Blutleitungssystem und ist schonend für im Blutleitungssystem befindliches Blut. Im Rahmen der Erfindung soll der Begriff "Anhalten" alle Möglichkeiten des Abschaltens, Stoppens und Leerlaufbetriebs einer Druckerzeugungseinrichtung umfassen, ohne dass diese Varianten jedes Mal differenziert erläutert werden müssten.

In einer Variante der Steuereinheit gemäß der Erfindung ist diese konfiguriert, zumindest eines der oben erläuterten Mittel, die geeignet sind, eine Einwirkung zumindest einer Druckerzeugungseinrichtung auf den Abschnitt zu beenden, wie etwa die zumindest eine Druckerzeugungseinrichtung oder zumindest ein Unterbrechungsmittel, anzusteuern, ohne vorher zu prüfen, ob eine Einwirkung vorliegt, und anschließend durch die Ansteuerung die Druckerzeugungseinrichtung anzuhalten und/oder das Unterbrechungsmittel zu schließen.

Wenn im Rahmen der Erfindung davon die Rede ist, dass die Steuereinheit oder eine andere geeignete Einheit etwas ausführt, ist dies eine vereinfachte Ausdrucksweise und dahingehend zu verstehen, dass die Steuereinheit oder die andere geeignete Einheit gegebenenfalls einen geeigneten Aktor oder Sensor ansteuert, etwas auszuführen, falls die Steuereinheit oder die andere geeignete Einheit nicht selbst zur direkten Ausführung in der Lage ist. Der Fachmann weiß in diesen Fällen, dass die entsprechende vereinfachte Formulierung entsprechend zu verstehen ist.

Vorteilhaft ist die Steuereinheit konfiguriert, die Schritte a) bis e) an einem arteriellen Abschnitt einer Blutzuführleitung des Blutleitungssystems und/oder an einem venösen Abschnitt einer Blutrückführleitung des Blutleitungssystems, insbesondere eines extrakorporalen Blutkreislauf, durchzuführen. Derartige Abschnitte können dadurch mit einem Blutgefäß in Fluidverbindung stehen, dass ein Ende des arteriellen und/oder venösen Abschnitts über einen Zugang, wie etwa eine Kanüle, in das Blutgefäß eingebracht ist. Ist das Blutgefäß eine arteriovenöse Fistel, kann eine arterielle Kanüle in einen arteriellen Abschnitt der Fistel eingebracht sein und/oder eine venöse Kanüle in einen venösen Abschnitt der Fistel eingebracht sein.

Der arterielle Abschnitt bzw. der venöse Abschnitt des Blutleitungssystems ist als derjenige Bereich des Blutleitungssystems definiert, der dem Blutgefäß, dessen Druck bestimmt werden soll, zugewandt ist. Der arterielle und der venöse Abschnitt können bei Vorliegen eines extrakorporalen Blutkreislaufs, beispielsweise in einer Hämodialysevorrichtung, an ihren vom Blutgefäß abgewandten Enden durch die Blutkammer eines Dialysators miteinander verbunden sein. Ein vom Blutgefäß abgewandtes Ende des venösen und/oder des arteriellen Abschnitts, dessen Druck in Schritt e) bestimmt wird, kann beispielsweise durch ein Unterbrechungsmittel und/oder eine Blutpumpe definiert sein. In der Blutzuführleitung kann beispielsweise eine peristaltische Pumpe als Druckerzeugungseinrichtung eingebracht sein und das vom Blutgefäß abgewandte Ende des arteriellen Abschnitts definieren.

Das Unterbrechungsmittel, das in Schritt b) von der Steuereinheit ansteuerbar ist, ist typischerweise an einem dem Blutgefäß zugewandten Ende des Abschnitts vorgesehen. Dadurch ist mit geringem Aufwand sicherzustellen, dass die Fluidverbindung des Blutleitungssystems mit dem Blutgefäß, dessen Druck bestimmt werden soll, unterbrochen werden kann.

Des Weiteren kann die Steuereinheit konfiguriert sein, zumindest ein weiteres Unterbrechungsmittel anzusteuern. Somit lässt sich beispielsweise die Fluidverbindung zwischen dem zumindest einen Abschnitt und dem restlichen Blutleitungssystem, insbesondere dem restlichen extrakorporalen Blutkreislauf, unterbrechen. Dies kann beispielsweise bei Vorliegen von zwei Abschnitten, deren Druck von der Steuereinheit gemäß der Erfindung in Schritt e) messbar ist, vorteilhaft sein. Wird in den zwei Abschnitten jeweils unabhängig voneinander und beispielsweise gleichzeitig in Schritt c) ein Druck-Sollwert eingestellt und in Schritt e) ein sich einstellender Druck gemessen, kann es vorteilhaft sein, wenn die beiden Abschnitte durch das weitere Unterbrechungsmittel bzw. die weiteren Unterbrechungsmittel voneinander getrennt sind, um ein Einstellen der jeweiligen Drücke ohne gegenseitige Beeinflussung der beiden Abschnitte zu gewährleisten. Dies kann insbesondere vorteilhaft sein, wenn die Fluidverbindung zumindest eines der beiden Abschnitte zum restlichen Blutleitungssystem hin anderweitig nicht oder nicht völlig unterbrechbar ist.

Als Unterbrechungsmittel in Schritt a), in Schritt b) und in Schritt d), sowie als weitere Unterbrechungsmittel können beispielsweise in einem extrakorporalen Blutkreislauf herkömmlich verwendete Unterbrechungsmittel, wie etwa Schlauchklemmen oder Ventile, von der Steuereinheit ansteuerbar sein.

Nach einer Ausführungsform kann die Steuereinheit konfiguriert sein, als Druckerzeugungseinrichtung eine Blutpumpe, insbesondere eine okkludierende Pumpe, wie etwa eine peristaltische Pumpe und/oder eine Rollenpumpe, anzusteuern. Peristaltische Pumpen, insbesondere Rollenpumpen, werden herkömmlich in Blutleitungssystemen in der Medizintechnik zum Fördern von Blut eingesetzt. Peristaltische Pumpen können vorwärts laufend betrieben werden und angehalten werden. Sie können des Weiteren auch geeignet sein, rückwärts laufend betrieben zu werden. Entsprechend kann die Steuereinheit konfiguriert sein. Denkbar ist auch, dass die Steuereinheit konfiguriert ist, die Pumpe in einen Leerlaufmodus zu versetzen, der trotz des Weiterlaufens der Pumpe eine Druckerzeugung verhindert. Durch ein Rückwärtslaufen der Blutpumpe wird nach einem Aspekt der Erfindung ein Unterdruck im Abschnitt verringert und auf den Sollwert, insbesondere Umgebungsdruck (entsprechend 0 mmHg), eingestellt.

Nach einer weiteren Ausführungsform kann die Steuereinheit auch konfiguriert sein, in Schritt a) und/oder Schritt c) alternativ oder zusätzlich zu einer Blutpumpe zumindest eine weitere Druckerzeugungseinrichtung, insbesondere Pumpe, anzusteuern, die dem extrakorporalen Blutkreislauf zugeordnet ist. Dies kann eine Substituatpumpe sein, die über eine Substituatleitung Substituat in die Blutzuführleitung (als sogenannte Prädilution) und/oder die Blutrückführleitung (als sogenannte Postdilution) pumpt.

Alternativ oder zusätzlich kann die Steuereinheit konfiguriert sein, in Schritt a) und/ oder Schritt c) als Druckerzeugungseinrichtung eine Pumpe in einem Dialysierflüssigkeitskreislauf anzusteuern, der mit dem Blutleitungssystem in Fluidverbindung steht. Dies kann insbesondere eine Ultrafiltrationspumpe sein. Dabei wirkt die Ultrafiltrationspumpe im Betrieb herkömmlich auf den extrakorporalen Blutkreislauf dadurch ein, dass über eine semipermeable Membran eines Dialysators, die eine Blutkammer von einer Dialysierflüssigkeitskammer trennt, eine Konvektion zur Dialysierflüssigkeitskammer hin entsteht. Diese Konvektion wirkt als Unterdruck auf die Blutkammer und damit den mit der Blutkammer verbundenen extrakorporalen Blutkreislauf ein. Durch ein Ansteuern und Abschalten bzw. Anhalten der Ultrafiltrationspumpe wird ebenfalls eine Druckerzeugung, die auf den zumindest einen Abschnitt einwirken kann, beendet. Wie die jeweiligen Pumpen im Dialysierflüssigkeitskreislauf, im Blutleitungssystem und/oder im Substituatleitungssystem bezüglich der Leitung, in die sie eingebracht sind, und ihrer genauen Funktionalität bei einer Blutbehandlung vorliegen, hat keinen Einfluss auf das Prinzip der Erfindung. Entsprechend sind die Schilderungen zur herkömmlichen Nutzung der Ultrafiltrationspumpe nicht einschränkend, sondern nur beispielhaft zu sehen. So kann beispielsweise die Ultrafiltrationspumpe auch nur den Teil der Ultrafiltration bewirken, der nicht von einer Substitutionsrate kompensiert wird.

Gemäß der Erfindung ist auch denkbar, dass die Steuereinheit konfiguriert ist, mehrere Druckerzeugungseinrichtungen anzusteuern, derart, dass diese in ihrer Summe einen Druck auf den zumindest einen Abschnitt ausüben, der gleich einem Sollwert, insbesondere gleich dem Umgebungsdruck ist.

Vorzugsweise werden jedoch in Schritt a) alle Druckerzeugungseinrichtungen, die auf den Abschnitt einwirken können, von der Steuereinheit jeweils derart angesteuert, dass sie keinen Druck auf den Abschnitt ausüben, indem sie beispielsweise angehalten werden. "Druck" wird im Rahmen der Erfindung übrigens stets als Oberbegriff von Überdruck und Unterdruck eingesetzt, wie bereits erläutert stets als Differenzdruck gegenüber dem Umgebungsdruck.

Nach einer weiteren Variante ist das Mittel, das ein Einwirken einer Druckerzeugungseinrichtung auf den Abschnitt verhindert bzw. beendet, eine Unterbrechungsvorrichtung, wie etwa eine Klemme oder ein Ventil, die trotz weiterlaufender Druckerzeugungseinrichtung bewirkt, dass die Druckerzeugungseinrichtung keinen Druck auf den Abschnitt mehr ausübt. Begrenzt beispielsweise eine venöse Klemme einen venösen Abschnitt des extrakorporalen Blutkreislaufs zum Dialysator hin und wirkt eine Ultrafiltrationspumpe über einen Dialysierflüssigkeitskreislauf, der über den Dialysator verläuft, auf den venösen Abschnitt ein, so wird die Einwirkung der Ultrafiltrationspumpe trotz ihres Weiterlaufens beendet, indem die Steuereinheit die Klemme schließt.

Die Steuereinheit kann konfiguriert sein, die Schritte a) bis e) in einer Blutbehandlungsvorrichtung, wie etwa einer Vorrichtung zur Hämodialyse, Hämofiltration und/oder Hämodiafiltration, durchzuführen. Bei dieser bevorzugten Variante der Erfindung sind eine Mehrzahl von Aktoren (insbesondere Druckerzeugungseinrichtungen und/oder Unterbrechungseinrichtungen) und Sensoren, die in Schritt a), b), c), d) und/oder e) von der Steuereinheit angesteuert werden können, bestehende Teile der Blutbehandlungsvorrichtung. Dies hat den Vorteil, dass die erfindungsgemäße Steuereinheit zumindest einen Teil der Schritte a) bis e), bevorzugt alle Schritte a) bis e), durchführen kann, ohne dass zusätzlich zu bereits in der Blutbehandlungsvorrichtung vorhandene Sensoren und Aktoren weitere Sensoren und/oder Aktoren nötig wären. Besonders bevorzugt sind alle Aktoren und Sensoren, die in Schritt a), b), c), d) und/oder e) von der Steuereinheit angesteuert werden können, Teile der Blutbehandlungsvorrichtung.

Selbstverständlich könnte die Steuereinheit im Rahmen der Erfindung aber auch konfiguriert sein, in einem oder mehreren der Schritte a) bis e) Aktoren und/oder Sensoren, wie etwa Pumpen, Ventile, Klemmen und/oder Drucksensoren, anzusteuern, die in der Blutbehandlungsvorrichtung ansonsten keine Aufgaben besitzen.

Nach einer weiteren bevorzugten Variante kann die erfindungsgemäße Steuereinheit konfiguriert sein, Sensoren und Aktoren in einer Blutbehandlungsvorrichtung im Rahmen einer Blutbehandlung anzusteuern. Dies hat den Vorteil, dass ein und dieselbe Steuereinheit genutzt werden kann, um die Blutbehandlungsvorrichtung zu steuern und eine Messung des Drucks in einem Blutgefäß vorzunehmen. Entsprechend verringern sich beispielsweise Kosten, Aufwand und Gewicht einer Kombination aus erfindungsgemäßer Steuereinheit und Blutbehandlungsvorrichtung.

Vorteilhaft kann die Steuereinheit konfiguriert sein, die Schritte a) bis e) zumindest einmal während einer Blutbehandlungssitzung durchzuführen. Die Angabe "während einer Blutbehandlungssitzung" umfasst gemäß der Erfindung beliebige Zeitpunkte im Rahmen einer Blutbehandlungssitzung. Diese können bei einer herkömmlicherweise mehrstündigen Blutbehandlung etwa ganz zu Beginn der Behandlung, ganz zum Ende der Behandlung oder zu einem Zeitpunkt zwischen Beginn und Ende der Behandlung liegen. Finden die Schritte a) bis e) ganz zu Beginn einer Blutbehandlungssitzung statt, ist es vorteilhaft, wenn zumindest die Blutzuführleitung bereits mit Blut des Patienten, dessen Blutgefäßdruck bestimmt werden soll, gefüllt ist. Ob die Blutzuführleitung mit Blut gefüllt ist, kann die Steuereinheit vorteilhafterweise vor Schritt a) kontrollieren. Damit ist gewährleistet, dass vor allem im Rahmen des Schritts e) keine Luft aus dem Blutleitungssystem in das Blutgefäßsystem des Patienten gelangt. Aber auch bei luftgefülltem Blutleitungssystem können die Schritte a) bis e) gemäß der Erfindung durchgeführt werden, denn auch mit Luft als Fluid kann eine Fluidverbindung des Abschnitts mit dem Blutgefäß vorliegen bzw. unterbrochen und wiederhergestellt werden.

Es kann des Weiteren vorgesehen sein, dass die Steuereinheit konfiguriert ist, die Schritte a) bis e) während einer Mehrzahl von Blutbehandlungssitzungen durchzuführen. Dadurch lässt sich eine langfristige Messreihe durchführen, um eine Entwicklung des gemessenen Drucks im Abschnitt und somit des Drucks im Blutgefäß, wie etwa des Fisteldrucks, zu beobachten.

Auch kann es vorteilhaft sein, dass die Steuereinheit konfiguriert ist, während einer Blutbehandlungssitzung die Schritte a) bis e) mehrfach durchzuführen. So lässt sich eine größere Anzahl von Messwerten auswerten, was zu besseren Genauigkeiten führen kann, beispielsweise durch Mittelwertbildung.

Selbstverständlich ist es auch möglich, die Schritte a) bis e) an einem Patienten durchzuführen, der zumindest zum Zeitpunkt der Messung des Drucks im Blutgefäß keiner Blutbehandlung unterzogen wird. Dies kann beispielsweise vorteilhaft sein, wenn eine noch größere Zahl von Messungen erhalten werden soll.

Nach einer Variante der Erfindung ist die Steuereinheit konfiguriert, von dem in Schritt e) gemessenen Druck den hydrostatischen Druck abzuziehen, der am Drucksensor anliegt. Dadurch wird der absolute Druck im Blutgefäß ermittelt, der nicht durch andere Drücke überlagert ist. Dies hat den Vorteil, dass bereits ein einziger Messwert eine hohe Aussagekraft hinsichtlich des Zustands des Blutgefäßes ermöglichen kann. Langzeitmessungen über mehrere Blutbehandlungssitzungen hinweg besitzen geringere Schwankungen im Kurvenverlauf und können zu exakteren Aussagen einer sich bildenden Stenose führen.

Die Subtraktion kann von der Steuereinheit beispielsweise in einer Auswerteinheit veranlasst werden. Eine Bestimmung des hydrostatischen Drucks und seine Übermittlung kann auf verschiedene Arten erfolgen. Der hydrostatische Druck p(hydr) berechnet sich nach der Formel

$$p(hydr) = \rho\ g\ h,$$

mit $\rho$ = Dichte von Blut,
g = Erdbeschleunigung (9,81 g/ms$^2$),
h = Höhendifferenz zwischen Drucksensor und Herz des Patienten

Nach einem Aspekt ist die Steuereinheit konfiguriert, beispielsweise über die Auswerteinheit die Höhendifferenz h abzufragen. Eine Eingabe kann manuell beispielsweise an einer mit der Auswerteinheit verbundenen Eingabeeinheit erfolgen oder durch Messung gewonnen werden.

Eine Messung kann durch Erfassung einer Lage des Patienten und damit seines Herzens erfolgen. Es können aber auch Schätzwerte, Näherungs- oder Erfahrungswerte genutzt werden, die beispielsweise patientenbezogen sein können. Diese können wiederum manuell eingegeben oder von der Auswerteinheit patientenbezogen eingesetzt werden.

Nach einem weiteren Aspekt der Erfindung ist die Steuereinheit konfiguriert, den in Schritt e) gemessenen Druck im Blutgefäß an eine Auswerteinheit zu übermitteln, die konfiguriert ist, eine Mehrzahl von Blutgefäßdruck-Werten hinsichtlich einer sich im Blutgefäß bildenden Stenose auszuwerten.

Insbesondere ist die Steuereinheit konfiguriert, einen in Schritt e) gemessenen Fisteldruck mit und/oder ohne überlagerten hydrostatischen Druck (d.h. den in Schritt e) gemessenen Druck im Abschnitt und/oder den berechneten Druck im Blutgefäß) an eine Auswerteinheit zu übermitteln, die konfiguriert ist, eine Mehrzahl von Druckwerten hinsichtlich einer sich bildenden Stenose im Blutgefäß, insbesondere einer Fistelstenose, auszuwerten.

Eine Auswertung einer Mehrzahl von Blutgefäßdruckwerten, insbesondere Fisteldruckwerten, kann aufgrund geeigneter Trendanalysen, also Langzeituntersuchungen, erfolgen. So kann die Steuereinheit selbst oder die Auswerteinheit konfiguriert sein, einen steigenden Druck im Blutgefäß als Zeichen einer sich bildenden Stenose zu erkennen. Stattdessen oder zusätzlich können sie konfiguriert sein, einen Abfall des Drucks im Blutgefäß, eine wachsende und/oder eine fallende erste Ableitung des Drucks als sich bildende Stenose zu erkennen. Je nach medizinischen Erkenntnissen über Stenosenbildung und ihre Auswirkungen auf den Druck im betroffenen Blutgefäß sind die verschiedensten Kriterien denkbar, aus dem Druckverlauf auf eine sich bildende Stenose zu schließen.

Bei Vorliegen eines vorbestimmten Kriteriums kann die Steuereinheit oder die Auswerteinheit eine Meldung abgeben. Diese Meldung kann beispielsweise über einen Signalgeber erfolgen. Entsprechend kann die Steuereinheit und/oder Auswerteinheit zum Geben der Meldung konfiguriert sein.

Die Aufgabe der Erfindung wird des Weiteren mit einem Verfahren gemäß Anspruch 13 gelöst. Gemäß einem erfindungsgemäßen Verfahren zur Bestimmung eines Drucks in einem Blutgefäß, insbesondere in einer arteriovenösen Fistel, das in Fluidverbindung mit zumindest einem Abschnitt eines Blutleitungssystems, insbesondere eines extrakorporalen Blutkreislaufs, steht, wobei zumindest eine Druckerzeugungseinrichtung vorgesehen ist, die geeignet ist, auf den Abschnitt einzuwirken, umfasst das Verfahren die folgenden Schritte:

> a) Sicherstellen, dass keine der zumindest einen Druckerzeugungseinrichtung auf den Abschnitt einwirkt,
> b) Unterbrechen der Fluidverbindung des Abschnitts mit dem Blutgefäß,
> c) Einstellen des Drucks in dem Abschnitt auf einen Sollwert, insbesondere auf Umgebungsdruck,
> d) Wiederherstellen der Fluidverbindung des Abschnitts mit dem Blutgefäß und
> e) Messen eines sich einstellenden Drucks im Abschnitt.

Vorteilhaft wird zumindest ein Teil der Schritte mit einer Steuereinheit durchgeführt. Dies kann vorzugsweise die erfindungsgemäße Steuereinheit sein. Es kann vorteilhaft sein, alle Schritte mit der Steuereinheit durchzuführen. Es kann aber auch gewünscht oder vorteilhaft sein, zumindest einen Teil der Schritte manuell durchzuführen. Insofern sollen alle Schritte und Teilschritte, die oben im Zusammenhang mit der Konfiguration der erfindungsgemäßen Steuereinheit erläutert wurden, als Verfahrensschritte offenbart gelten, die von der Steuereinheit ausgeführt werden können, aber auch anderweitig, insbesondere manuell, ausgeführt werden können, ohne dass dies im Folgenden für jede einzelne Variante und Alternative der Schritte a) bis e) explizit ausgeführt wird. Dasselbe gilt für alle weiteren Schritte und Teilschritte im Rahmen der Erfindung.

Die Aufgabe, die sich die Erfindung gestellt hat, wird des Weiteren mit einer Blutbehandlungsvorrichtung nach Anspruch 15 gelöst.

Eine erfindungsgemäße Blutbehandlungsvorrichtung zur extrakorporalen Blutbehandlung, insbesondere zur Hämodialyse, Hämofiltration und/oder Hämodiafiltration, ist vorgesehen zur Aufnahme eines Blutleitungssystems eines extrakorporalen Blutkreislaufs, wobei das Blutleitungssystem einen arteriellen Abschnitt einer Blutzuführleitung und einen venösen Abschnitt einer Blutrückführleitung besitzt. Der arterielle Abschnitt und/oder der venöse Abschnitt sind dafür vorgesehen, mit einem Blutgefäß, insbesondere einer arteriovenösen Fistel, in Fluidverbindung gesetzt zu werden, und die Blutbehandlungsvorrichtung weist zumindest eine Druckerzeugungseinrichtung auf, die geeignet ist, auf den arteriellen und/oder den venösen Abschnitt einzuwirken. Des Weiteren weist die Blutbehandlungsvorrichtung eine erfindungsgemäße Steuereinheit zur Bestimmung eines Drucks in dem Blutgefäß auf.

Im Folgenden werden die Erfindung und weitere vorteilhafte Varianten und Ausführungsformen anhand eines Ausführungsbeispiels unter Bezugnahme auf die Figur näher beschrieben.

Die Figur zeigt schematisch den Aufbau einer Hämodiafiltrationsvorrichtung (HDF-Vorrichtung) zusammen mit einer erfindungsgemäßen Steuereinheit zur Bestimmung eines Drucks in einem Blutgefäß. Die HDF-Vorrichtung lässt sich auch als HD-Vorrichtung auffassen, solange keine Hämofiltration durchgeführt wird, bzw. als HF-Vorrichtung, solange keine Hämodialyse damit durchgeführt wird. Die Blutbehandlungsvorrichtung im Ausführungsbeispiel kann daher je nachdem, welche Art der Blutbehandlung gerade besprochen wird, auch entsprechend benannt sein. Die Bezeichnungen HD, HF bzw. HDF sind dabei jeweils nicht beschränkend zu sehen.

Anhand der Figur werden zunächst der prinzipielle Aufbau eines Hämodiafiltrationsgeräts und seine Anbindung an das lediglich angedeutete Blutgefäßsystem I eines (nicht dargestellten) Patienten kurz erläutert. Bei der Hämodialyse wird Blut aus dem Blutgefäßsystem I in einen extrakorporalen Blutkreislauf II geleitet. Zu diesem Zweck ist dem Patienten eine Fistel F gelegt, die einen Kurzschluss zwischen einer Arterie A und einer Vene V beispielsweise im linken Unterarm (nicht dargestellt) bildet und somit eine sogenannte arteriovenöse Fistel darstellt. Über eine arterielle Kanüle 1 ist eine Blutzuführleitung 2 mit der Fistel F verbunden. Blut aus dem Blutgefäßsystem I wird mittels einer typischerweise als okkludierende Rollenpumpe ausgebildeten Blutpumpe 3 über die Blutzuführleitung 2 einem als Hämodialysator 4 ausgeführten Blutreinigungselement zugeführt. Im Hämodialysator 4 trennt eine vorzugsweise in Form einer Vielzahl von nicht dargestellten Hohlfasern ausgebildeten semipermeablen Membran 5 eine auch als Blutkammer bezeichnete erste Kammer 6, die Teil des extrakorporalen Blutkreislaufs II ist, von einer auch als Dialysierflüssigkeitskammer bezeichneten zweiten Kammer 7, die Teil eines Dialysierflüssigkeitskreislaufs III ist. Durch die semipermeable Membran 5 treten aus dem Blut zu entfernende Stoffe in die Dialysierflüssigkeit über, die durch die Dialysierflüssigkeit abgeführt werden. Gleichzeitig kann über einen Druckgradienten eine überschüssige Flüssigkeitsmenge aus dem Blut ultrafiltriert werden und ebenfalls über die abfließende Dialysierflüssigkeit entfernt werden. Der Druckgradient wird durch eine Ultrafiltrationspumpe 8 erzeugt.

Das gereinigte Blut verlässt die Blutkammer 6 des Hämodialysators 4 über eine Blutrückführleitung 9 und wird über eine venöse Kanüle 10, die in einen der Vene V des Patienten zugewandten Teil der Fistel F eingestochen ist, in das Blutgefäßsystem I des Patienten zurückgeführt. An der Blutrückführleitung 9 ist eine venöse Klemme 11 als venöse Unterbrechungseinrichtung vorgesehen, mit der die Rückführung von Blut beispielsweise in Notfällen unterbrochen werden kann. Derartige Notfälle können z.B. auftreten, wenn durch einen Luftdetektor 12 zwischen Dialysator 4 und venöser Klemme 11 Luft in der Blutrückführleitung 9 detektiert wird. An der Blutzuführleitung 2 ist stromauf der Blutpumpe 3 ein arterieller Drucksensor 13 und an der Blutrückführleitung 9 stromauf der venösen Klemme 11 ein venöser Drucksensor 14 vorgesehen.

Die zweite Kammer 7 des Hämodialysators 4 wird von Dialysierflüssigkeit durchströmt, die über eine Dialysierflüssigkeitszuführleitung 15 von einer Dialysierflüssigkeitsquelle 16 zugeführt und über eine Dialysierflüssigkeitsabführleitung 17 zu einem Abfluss 18 abgeführt wird. Die Dialysierflüssigkeit wird durch eine Dialysierflüssigkeitspumpe 19 in der Dialysierflüssigkeitsabführleitung 17 gefördert. Stromauf der Dialysierflüssigkeitspumpe 19 zweigt von der Dialysierflüssigkeitsabführleitung 17 eine Ultrafiltratleitung 20 ab, in die die Ultrafiltrationspumpe 8 geschaltet ist und die auch zum Abfluss 18 führt.

Um dem Patienten wieder Flüssigkeit zuzuführen, verfügt das HDF-Gerät über eine Substitutionseinrichtung 21, mit der dem Blut im extrakorporalen Blutkreislauf II eine Substitutionsflüssigkeit (auch als Substituat bezeichnet) zugeführt werden kann. Die Substitutionseinrichtung 21 weist eine Substituatquelle 22 zur Bereitstellung von Substituat auf, von der eine erste Substituatleitung 23, in die eine erste Substituatpumpe 24 geschaltet ist, stromab der Blutpumpe 3 in die Blutzuführleitung 2 führt, was als Prädilution bezeichnet wird, da die Substituatzuführung vor der Blutkammer 6 erfolgt. Von der Substituatquelle 22 führt eine zweite Substituatleitung 25, in die eine zweite Substituatpumpe 26 geschaltet ist, stromab der Blutkammer 6 (Postdilution) in die Blutrückführleitung 9. Die zweite Substituatleitung 25 mündet in die Tropfkammer 12 der Rückführleitung 9.

Verschiedene Bilanziereinrichtungen ermöglichen, die Menge an Substituat und Dialysierflüssigkeit, die zugeführt werden, und die Menge an Ultrafiltrat sowie Dialysierflüssigkeit, die abgeführt werden, im Zusammenspiel mit den genannten und eventuell zusätzlichen Pumpen gezielt aufeinander abzustimmen. Dem Fachmann stehen zur Realisierung einer Bilanziereinrichtung 27, die zugeführte Dialysierflüssigkeit und abgeführte Dialysierflüssigkeit bilanziert, und eventueller weiterer Bilanziereinrichtungen und Pumpen im Dialysierflüssigkeitskreislauf und bei der Substitutionseinrichtung verschiedenste Ausgestaltungen zur Verfügung, so dass an dieser Stelle von detaillierten Ausführungen abgesehen wird. Gleiches gilt auch für die Bereitstellung von Dialysierflüssigkeit durch die Dialysierflüssigkeitsquelle 16 und von Substituat durch die Substituatquelle 22.

Auch zum Einsatz von Aktoren und Sensoren in einem HDF-Gerät im Allgemeinen stehen dem Fachmann zahlreiche Möglichkeiten zur Verfügung, ohne dass hier im Detail auf alle diese Möglichkeiten eingegangen werden muss. Die Darstellung in der Figur ist auf einige wenige dieser Aktoren und Sensoren beschränkt, die für die Erläuterung der Erfindung ausreichend sind, wie etwa die venöse Klemme 11, den arteriellen Drucksensor 13 und die Ultrafiltrationspumpe 8.

Das HDF-Gerät wird durch eine Steuereinheit 30 gesteuert und überwacht. Hierzu ist die Steuereinheit 30 mit den einzelnen Aktoren und Sensoren des Gerätes mit Signalleitungen verbunden. Für die in der Figur dargestellten Aktoren und Sensoren, wie etwa Pumpen, Drucksensoren, Klemmen und Ventile, ist dies durch eine Mehrzahl von Signalleitungen 50 lediglich pauschal angedeutet, die für die einzelnen Aktoren oder Sensoren wegen der sich sonst ergebenden schlechten Übersichtlichkeit nicht einzeln dargestellt und nicht mit einem individuellen Bezugszeichen bezeichnet sind.

Die erfindungsgemäße Steuereinheit zur Bestimmung des Drucks in einem Blutgefäß wird im Zusammenspiel mit der eben beschriebenen Hämodiafiltrationsvorrichtung erläutert, da in dieser die meisten oder sogar alle gemäß der Erfindung angesteuerten Hardware-Komponenten, insbesondere Aktoren und Sensoren, bereits vorhanden sind. Die Erfindung beschränkt sich aber nicht auf die Anwendung der Steuereinheit in der konkret beschriebenen HDF-Vorrichtung. Die Steuereinheit kann Bestandteil des HDF-Geräts sein oder eine separate Einheit bilden, die an ein bestehendes HDF-Gerät anzuschließen ist. Entsprechendes gilt aber für jede andere Blutbehandlungsvorrichtung, wie etwa ein Hämofiltrationsgerät und ein Hämodialysegerät, an die eine erfindungsgemäße Steuereinheit angeschlossen werden kann.

Die im Folgenden als von der Steuereinheit ausgeführt erläuterten Verfahrensschritte können des Weiteren entweder allesamt von der erfindungsgemäßen Steuereinheit gesteuert werden oder im Rahmen des erfindungsgemäßen Verfahrens wahlweise zumindest teilweise manuell ausgeführt werden, bzw. von weiteren Einrichtungen, wie einer Auswerteinheit, einer Speichereinheit, einer Eingabeeinheit, einem Signalgeber oder weiteren Einrichtungen, die ihrerseits Schritte nach Ansteuerung durch die Steuereinheit oder manuell bedient oder selbstständig ausführen, ausgeführt werden.

Wenn im Folgenden davon die Rede ist, dass die Steuereinheit oder eine andere geeignete Einheit etwas "ausführt", beispielsweise einen Druck misst oder eine Klemme schließt, ist dies eine vereinfachte Ausdrucksweise und dahingehend zu verstehen, dass die Steuereinheit oder die andere geeignete Einheit einen geeigneten Aktor oder Sensor gegebenenfalls nach Abfrage eines Status ansteuert, etwas auszuführen, beispielsweise einen Drucksensor ansteuert, einen Druck zu messen und den gemessenen Druck an die Steuereinheit zu melden, oder eine Klemme ansteuert, zu schließen, eventuell nach Abfrage, ob diese bereits geschlossen ist, etc. Der Einfachheit halber wird nicht in allen Fällen ausformuliert, welcher Aktor oder Sensor nach einer Ansteuerung aktiv wird. Der Fachmann weiß in diesen Fällen, wie die entsprechende vereinfachte Formulierung zu verstehen ist.

Beim Ausführungsbeispiel der Erfindung ist die Steuereinheit 30 konfiguriert, in einem Schritt a) sicherzustellen, dass die Blutpumpe nicht mehr auf einen ersten Abschnitts 28 der Blutzuführleitung 2 einwirkt, der sich von der Blutpumpe 3 bis zu einer arteriellen Klemme 29, die die Blutzuführleitung 2 zum Patienten hin schließen kann, erstreckt, und der im Folgenden auch kurz "arterieller Abschnitt" genannt wird. Dazu kann die Steuereinheit 30 die Blutpumpe 3 ansteuern, dass diese in Schritt a) stoppt. Dadurch endet die Erzeugung eines Unterdrucks im arteriellen Abschnitt. Des Weiteren ist die Steuereinheit 30 konfiguriert, in einem Schritt b) eine Fluidverbindung des arteriellen Abschnitts von der arteriovenösen Fistel durch Ansteuerung eines Unterbrechungsmittels zu unterbrechen. In diesem Beispiel ist sie dazu konfiguriert, die Fluidverbindung von der arteriovenösen Fistel F zu unterbrechen, indem die arterielle Klemme 29 als Unterbrechungsmittel angesteuert und geschlossen wird.

Die Steuereinheit 30 ist des Weiteren konfiguriert, die Blutpumpe 3 in einem Schritt c) als Druckerzeugungsmittel so anzusteuern, dass im arteriellen Abschnitt 28 ein Soll-Wert des Drucks von 0 mmHg erzielt wird, d.h. Umgebungsdruck. Insbesondere ist die Steuereinheit konfiguriert, die Blutpumpe 3 je nach Bedarf vorwärts oder rückwärts laufen lassen. Die Steuereinheit 30 ist konfiguriert, vom arteriellen Drucksensor 13 den Druck im arteriellen Abschnitt 28 abzufragen und im Falle, dass dieser positiv ist, die Blutpumpe vorwärts laufen zu lassen, so dass stromauf der Blutpumpe 3 ein negativer Druck erzeugt wird. Auch ist die Steuereinheit 30 so konfiguriert, dass sie im Falle, dass der Druck im arteriellen Abschnitt 28 negativ ist, die Blutpumpe 3 so ansteuern kann, dass diese rückwärts läuft und so einen positiven Druck erzeugt, der den negativen Druck bis auf Null kompensiert. Mit den Laufrichtungen "vorwärts" und "rückwärts" sind die Richtungen bezeichnet, die sich auf den oben geschilderten normalen Hämodialysebetrieb beziehen. Um die anhand der Figur geschilderten Blutflüsse im extrakorporalen Blutkreislauf im Normalbetrieb von der arteriellen Kanüle 1 über den Dialysator 4 zurück zur venösen Kanüle 10 zu erzielen, läuft die Blutpumpe 3 per Definition "vorwärts".

Die Steuereinheit ist außerdem konfiguriert, in einem Schritt d) die Fluidverbindung des ersten arteriellen Abschnitts 28 mit der arteriovenösen Fistel F wiederherzustellen, indem die arterielle Klemme 29 nach ihrer Ansteuerung wieder öffnet. Weiters ist die Steuereinheit konfiguriert, in einem Schritt e) einen sich einstellenden Druck im Abschnitt 28 mittels des arteriellen Drucksensors 13 zu messen.

Die weiteren Konfigurationen der Steuereinheit werden im Folgenden im Rahmen des erfindungsgemäßen Verfahrens geschildert.

Gemäß dem Ausführungsbeispiel des erfindungsgemäßen Verfahrens befindet sich der Patient zunächst in einem laufenden Hämodiafiltrationsverfahren. Das heißt, die Blutpumpe 3 pumpt Blut aus der Fistel F durch die Blutzuführleitung 2 in die erste Kammer 6 des Hämodialysators 4 und über die Blutrückführleitung 9 und die venöse Kanüle 10 zurück in die Fistel F. Die venöse Klemme 11 und die arterielle Klemme 29 in der Blutzuführleitung als arterielle Unterbrechungseinrichtung sind geöffnet. Durch den Hämodialysator 4 werden dem Blut unerwünschte Bestandteile entzogen und das Blut dadurch gereinigt. Der arterielle Drucksensor 13 misst einen Druck im arteriellen Abschnitt und der venöse Drucksensor 14 einen Druck im venösen Abschnitt. Diese Drücke setzen sich zusammen aus dem Staudruck an der arteriellen bzw. der venösen Kanüle, dem hydrostatischen Druck und dem Fisteldruck. Der hydrostatische Druck entsteht durch die Flüssigkeitssäule aus Blut bis zur Höhe des Herzens des Patienten, die in den Blutgefäßen des Patienten oberhalb des arteriellen bzw. venösen Drucksensors steht, und ist von der Position und Lage des Patienten abhängig. Der Fisteldruck, auch als Fistelinnendruck bezeichnet, ist der Druck in der Fistel, der letztendlich aus dem Druck des Herzschlages als Staudruck in der Fistel resultiert.

Nun wird die Blutpumpe 3 angehalten und sowohl die arterielle als auch die venöse Klemme 29, 11 wird geschlossen. Der arterielle Drucksensor 13 misst nun einen arteriellen Druck. Wegen des Stillstands der Blutpumpe 3 entfällt der Staudruck. Dabei weist der gemessene Druck einen gewissen zeitlichen Verlauf auf, denn der Staudruck fällt nicht schlagartig nach Abschalten der Blutpumpe ab, sondern verebbt im Lauf einiger Sekunden. Durch das Schließen der Klemmen 29, 11 wird der vorliegende Druckzustand quasi "eingefroren", d.h. vom Blutgefäßsystem I des Patienten entkoppelt.

Nun wird der Druck in der Blutzuführleitung auf 0 mmHg, d.h. auf Umgebungsdruck, tariert. Dies wird bei weiterhin geschlossener arterieller Klemme 29 dadurch erreicht, dass mit den vorhandenen Aktoren ein Druck bzw. Unterdruck erzeugt wird, der den bestehenden Druck im Abschnitt, dessen Druck tariert wird, vollständig kompensiert. Dies wird in der Blutzuführleitung 2 mit der Blutpumpe 3 erreicht.

Bei Vorliegen eines negativen Ausgangsdrucks in dem ersten arteriellen Abschnitt der Blutzuführleitung 2 zwischen Blutpumpe 3 und arterieller Klemme 29 von beispielsweise - 180 mmHg, wie er durch die Saugwirkung stromauf der Blutpumpe 3 bei einer Blutbehandlung typischerweise entstehen kann, wird die Blutpumpe 3 in einem Schritt c) entgegen der herkömmlichen Pumprichtung betrieben, so dass ein positiver Druck erzeugt wird. Die Blutpumpe 3 wird solange laufen gelassen, bis sich ein Druck von 0 mmHg einstellt. Dann wird die Blutpumpe 3 angehalten.

Liegt stattdessen ein positiver Ausgangsdruck im ersten arteriellen Abschnitt vor, den der arterielle Drucksensor 13 misst, wird die Blutpumpe in Schritt c) in ihrer herkömmlichen Laufrichtung betrieben. Der stromab der Blutpumpe 3 dadurch erzeugte Unterdruck gleicht den positiven Ausgangsdruck entsprechend solange aus, bis der Wert 0 mmHg, also Umgebungsdruck, erzielt wird. Dann wird die Blutpumpe 3 angehalten.

Dadurch, dass die Blutpumpe 3 als peristaltische Pumpe punktuell den Schlauch okkludiert, der im Bereich der Pumpe den dortigen Abschnitt des Blutleitungssystems bildet, findet kein Druckausgleich stromauf und stromab der Blutpumpe statt. Dadurch lässt sich der gewünschte Druck von 0 mmHg in diesem Beispiel exakt einstellen, da der erste arterielle Abschnitt während des Schritts c) ein geschlossenes System darstellt.

Nun wird in einem Schritt d) die arterielle Klemme 29 geöffnet, wodurch die Fluidverbindung zur Fistel F wiederhergestellt wird. Es stellt sich ein Druck p(art) im arteriellen Abschnitt 28 ein, der vom arteriellen Drucksensor 13 gemessen wird. Der Druck im arteriellen Abschnitt setzt sich aus dem Fisteldruck und dem hydrostatischen Druck zusammen. Als Fisteldruck wird dabei der mittlere Druck in der Fistel bezeichnet. Durch die Herzschläge des Patienten ergibt sich ein wellenförmiger Druckverlauf, der über die Blutgefäße und das Blutleitungssystem, insbesondere auch durch die Verengung der Kanülen 1, 10 gedämpft wird. Daher wird die Verwendung des Mittelwerts des Drucks im zu vermessenden Blutgefäß (hier der Fistel) bevorzugt.

Mit Kenntnis der Lage des Herzens des Patienten lässt sich der hydrostatische Druck p(hydr) mit

$$P(hydr) = \rho \; g \; h$$

(mit $\rho$ = Dichte des Bluts, g = Erdbeschleunigung, h= Höhe des Herzens über dem Drucksensor 13) berechnen und von p(art) abziehen, wodurch man den Fisteldruck p(Fistel) erhält:

$$p(Fistel) = p(art) - p(hydrostat)$$

Diese Berechnung wird in diesem Ausführungsbeispiel von einer Auswerteinheit 34 ausgeführt. Dazu misst das HDF-Gerät bedienendes medizinisches Personal die Höhe des Herzens des Patienten und gibt sie in eine Eingabeeinheit ein, hier einen Touchscreen 33 ein, wenn die Auswerteinheit 30 sie abfragt. Der sich ergebende absolute Fisteldruck ohne überlagerten hydrostatischen Druck wird von der Auswerteinheit wiedergegeben, im Bildschirm 33 angezeigt, und in der Auswerteinheit 34 gespeichert.

Im Ausführungsbeispiel wird die beschriebene Bestimmung des Fisteldrucks bei jeder einzelnen Blutbehandlungssitzung des Patienten durchgeführt, also typischerweise dreimal pro Woche. Die dabei bestimmten Werte des Fisteldrucks werden von der Auswerteinheit datumsbezogen gespeichert und als zeitabhängige Messkurve in einem Diagramm aufgetragen. Überschreitet der Fisteldruck einen vorbestimmten Wert, wird ein Warnhinweis gegeben, der beispielsweise am Bildschirm 33 angezeigt wird. Stattdessen kann eine Warnung auch bei einem charakteristischen Kurvenverlauf der zeitabhängigen Messkurve gegeben werden. Je nachdem wo sich in oder an der Fistel eine Stenose bildet, können andere Druckverläufe typisch sein. Dies kann beispielsweise ein Überschreiten oder Unterschreiten der ersten Ableitung über einen vorbestimmten Wert sein. Vorbestimmte Werte des Drucks, dessen Ableitung oder anderer vorbestimmte Kurvenparameter, können dabei für jeden Patienten individuell vorbestimmt werden, beispielsweise in Abhängigkeit einer vorliegenden Hypertonie, einer vorliegenden Kalzifizierung der Blutgefäße oder anderer Vorerkrankungen, die den Fisteldruck und/oder dessen zeitlichen Verlauf beeinflussen könnten.

Besonders Stenosen im abführenden Teil der Fistel, sogenannte Abflussstenosen, lassen sich anhand einer charakteristischen Fisteldrucksteigerung mit dem erfindungsgemäßen Verfahren leicht erkennen.

In einer Variante des Ausführungsbeispiels ist die Steuereinheit 30 konfiguriert, in Schritt a) die Ultrafiltrationspumpe 8 anzusteuern und anzuhalten. Des Weiteren ist die Steuereinheit 30 konfiguriert, in Schritt b) einen im Folgenden auch kurz "venöser Abschnitt 41" genannten Abschnitt 41 der Blutrückführleitung 9 zwischen der venösen Klemme 11 und einer stromab der Blutkammer 6 angeordnete Klemme 42 von einer Fluidverbindung von der Fistel F dadurch zu unterbrechen, dass als Unterbrechungsmittel die venöse Klemme 11 angesteuert wird. Anschließend wird in Schritt c) durch die Ultrafiltrationspumpe 8 der ursprünglich im Normalbetrieb bei ca. +200 mmHg liegende Druck im venösen Abschnitt solange verringert, bis der Wert 0 mmHg, also Umgebungsdruck, erreicht ist. Dann wird die Ultrafiltrationspumpe angehalten. In Schritt d) wird wiederum die Fluidverbindung hergestellt, in der Variante durch Ansteuern der venösen Klemme 11. Der in Schritt e) gemessene Wert ist in der Variante der mittels des venösen Drucksensors 14 gemessene Druck im venösen Abschnitt 41.

In einer weiteren Variante des Ausführungsbeispiels ist mindestens eine weitere Pumpe vorgesehen, um ein Tarieren mindestens eines der Drücke in der Blutzuführleitung und/oder der Blutrückführleitung durchzuführen. In dieser Variante ist die Steuereinheit 30 dafür ausgebildet, diese mindestens eine weitere Pumpe zu steuern. Die mindestens eine weitere Pumpe kann eine weitere Pumpe sein, die bei der Hämodialyse bereits eine andere Aufgabe erfüllt, wie etwa eine der beiden Substituatpumpen 24, 26. Durch Pumpen von Substituat in das Blutleitungssystem 39 lässt sich der Druck darin erhöhen. Insbesondere in Kombination mit der Ultrafiltrationspumpe kann hier ein Tarieren des Drucks im arteriellen und/oder venösen Abschnitt 28, 41 erzielt werden.

Bei einer Blutbehandlungsvorrichtung gemäß dem Ausführungsbeispiel wird eine oben erläuterte Erkennung eines kritischen Fisteldrucks mittels der Steuereinheit 30 durchgeführt.

Die erfindungsgemäße Vorrichtung zur Durchführung des Verfahrens enthält in dem Ausführungsbeispiel eine Steuereinheit 30, die dazu ausgebildet ist, die oben erläuterten Rechenschritte zu Bestimmung des Fisteldrucks durchzuführen, sowie die Klemmen 11, 29, 42 wie geschildert zu schließen und zu öffnen. Des Weiteren ist die Steuereinheit 30 dazu ausgebildet und vorgesehen, die Blutpumpe 3 und die Ultrafiltrationspumpe 39 so anzusteuern, dass die erläuterten Drücke eingestellt werden können.

In einer weiteren Variante des Ausführungsbeispiels wird der hydrostatische Druck nicht vom gemessenen Druck im arteriellen und/oder venösen Abschnitt abgezogen. In diesem Fall wird der Fisteldruck als Summe mit dem hydrostatischen Druck bestimmt. Langfristige Tendenzen eines steigenden Fisteldrucks werden aber festgestellt, obwohl der Absolutwert des Fisteldrucks nicht berechnet wird, denn Steigungen und andere Trends sind nicht vom Absolutwert des Drucks abhängig. Insbesondere, wenn der Patient während der Hämodialyse hinsichtlich Position und Lage bei jeder erfindungsgemäßen Messung weitestgehend identisch angeordnet ist, hat der hydrostatische Druck nur wenig Einfluss auf die Genauigkeit der Messung.

Die Erfindung ist nicht auf die beschriebenen Ausführungsbeispiele beschränkt. Insbesondere kann jede Art von Pumpe, die zum Pumpen von Blut bzw. zur Erzeugung eines Drucks geeignet ist, sowohl in der Blutzuführleitung als auch der Blutrückführleitung vorgesehen sein. Außerdem können alle genannten Merkmale beliebig miteinander kombiniert werden, solange dies im Rahmen der Erfindung sinnvoll und machbar ist. Einzelne Schritte oder Unterschritte des Verfahrens können alle von der Steuereinheit ausgeführt werden oder zumindest teilweise manuell.

## Patentansprüche

1. Steuereinheit (30) zur Bestimmung eines Drucks in einem Blutgefäß (A, V, F), insbesondere in einer arteriovenösen Fistel (F), das in Fluidverbindung mit zumindest einem Abschnitt (28, 41) eines Blutleitungssystems (39), insbesondere eines extrakorporalen Blutkreislaufs (II), steht, wobei dem Blutleitungssystem (39) zumindest eine Druckerzeugungseinrichtung (3, 8, 19, 24, 26) zugeordnet ist, die geeignet ist, auf den Abschnitt (28, 41) einzuwirken, und wobei die Steuereinheit (30) konfiguriert ist, die folgenden Schritte durchzuführen:

   a) Sicherstellen, dass keine der zumindest einen Druckerzeugungseinrichtung (3, 8, 19, 24, 26) auf den Abschnitt (28, 41) einwirkt,
   b) Unterbrechen der Fluidverbindung des Abschnitts (28, 41) mit dem Blutgefäß (A, V, F) durch Ansteuern eines Unterbrechungsmittels (11, 29),
   c) Einstellen des Drucks im Abschnitt (28, 41) auf einen vorbestimmten Sollwert, insbesondere auf den Umgebungsdruck, mit Hilfe eines Drucksensors (13, 14) im Abschnitt (28, 41),
   d) Wiederherstellen der Fluidverbindung des Abschnitts (28, 41) mit dem Blutgefäß (A, V, F) durch Ansteuern des Unterbrechungsmittels (11, 29) und
   e) Messen eines sich einstellenden Drucks im Abschnitt (28, 41) mit Hilfe des Drucksensors (13, 14).

2. Steuereinheit (30) nach Anspruch 1, die konfiguriert ist, die Schritte a) bis e) an einem arteriellen Abschnitt (28) einer Blutzuführleitung (2) und/oder an einem venösen Abschnitt (41) einer Blutrückführleitung (9) des Blutleitungssystems (39) durchzuführen.

3. Steuereinheit (30) nach Anspruch 1 oder 2, die konfiguriert ist, Schritt a) dadurch auszuführen, dass die zumindest eine Druckerzeugungseinrichtung (3, 8, 19, 24, 26) angesteuert wird.

4. Steuereinheit (30) nach zumindest einem der vorhergehenden Ansprüche, die konfiguriert ist, als Druckerzeugungseinrichtung (3, 8, 19, 24, 26) eine Blutpumpe (3), insbesondere eine peristaltische Pumpe (3) anzusteuern.

5. Steuereinheit (30) nach Anspruch 4, die konfiguriert ist, die Blutpumpe (3) derart anzusteuern, dass sie rückwärts läuft und dadurch einen Unterdruck im Abschnitt (28, 41) verringert und auf den Sollwert, insbesondere den Umgebungsdruck erhöht.

6. Steuereinheit nach zumindest einem der vorhergehenden Ansprüche, die konfiguriert ist, in Schritt a) und/oder c) als Druckerzeugungseinrichtung eine Pumpe, insbesondere eine Ultrafiltrationspumpe (8), in einem Dialysierflüssigkeitskreislauf (III) anzusteuern, der mit dem Blutleitungssystem (39) in Fluidverbindung steht.

7. Steuereinheit nach zumindest einem der vorhergehenden Ansprüche, die konfiguriert ist, die Schritte a) bis e) in einer Blutbehandlungsvorrichtung, wie etwa einer Vorrichtung zur Hämodialyse, Hämofiltration und/oder Hämodiafiltration durchzuführen.

8. Steuereinheit nach zumindest einem der vorhergehenden Ansprüche, die konfiguriert ist, die Schritte a) bis e) zumindest einmal während einer Blutbehandlungssitzung durchzuführen.

**9.** Steuereinheit nach zumindest einem der vorhergehenden Ansprüche, die konfiguriert ist, die Schritte a) bis e) während einer Mehrzahl von Blutbehandlungssitzungen durchzuführen.

**10.** Steuereinheit nach zumindest einem der vorhergehenden Ansprüche, die konfiguriert ist, zumindest ein weiteres Unterbrechungsmittel (42, K2) anzusteuern, um die Fluidverbindung zwischen dem zumindest einen Abschnitt (28, 41) und dem restlichen Blutleitungssystem (39) zu unterbrechen.

**11.** Steuereinheit nach zumindest einem der vorhergehenden Ansprüche, die konfiguriert ist, eine Auswerteinheit (34) anzusteuern, dass die Auswerteinheit (34) einen am Drucksensor (13, 14) anliegenden hydrostatischen Druck vom in Schritt e) gemessenen Druck im Abschnitt (28, 41) abzieht.

**12.** Steuereinheit nach zumindest einem der vorhergehenden Ansprüche, die konfiguriert ist, den Druck im Blutgefäß (A, V, F) und/oder den in Schritt e) gemessenen Druck im Abschnitt (28, 41) an eine Auswerteinheit (34) zu übermitteln, die konfiguriert ist, eine Mehrzahl von Druckwerten hinsichtlich einer sich bildenden Stenose auszuwerten.

**13.** Verfahren zur Bestimmung eines Drucks in einem Blutgefäß (A, V, F), insbesondere in einer arteriovenösen Fistel (F), das in Fluidverbindung mit zumindest einem Abschnitt (28, 41) eines Blutleitungssystems (39), insbesondere eines extrakorporalen Blutkreislaufs (II), steht, wobei zumindest eine Druckerzeugungseinrichtung (3, 8, 19, 24, 26) vorgesehen ist, die geeignet ist, auf den Abschnitt (28, 41) einzuwirken, wobei das Verfahren die folgenden Schritte aufweist:

a) Sicherstellen, dass keine der zumindest einen Druckerzeugungseinrichtung (3, 8, 19, 24, 26) auf den Abschnitt (28, 41) einwirkt,
b) Unterbrechen der Fluidverbindung des Abschnitts (28 41) mit dem Blutgefäß (A, V, F),
c) Einstellen des Drucks in dem Abschnitt auf einen Sollwert, insbesondere auf Umgebungsdruck,
d) Wiederherstellen der Fluidverbindung des Abschnitts (28, 41) mit dem Blutgefäß (A, V, F) und
e) Messen eines sich einstellenden Drucks im Abschnitt (28, 41).

**14.** Verfahren nach Anspruch 13, **dadurch gekennzeichnet, dass** zumindest einer der Schritte a) bis e) von einer Steuereinheit (30) durchgeführt wird.

**15.** Blutbehandlungsvorrichtung zur extrakorporalen Blutbehandlung, insbesondere zur Hämodialyse, Hämofiltration und/oder Hämodiafiltration, vorgesehen zur Aufnahme eines Blutleitungssystems (39) eines extrakorporalen Blutkreislaufs (II), wobei das Blutleitungssystem (39) einen arteriellen Abschnitt (28) einer Blutzuführleitung (2) und/oder einen venösen Abschnitt (41) einer Blutrückführleitung (9) besitzt, wobei der arterielle Abschnitt (28) und der venöse Abschnitt (41) dafür vorgesehen sind, mit einem Blutgefäß (A, V, F), insbesondere einer arteriovenösen Fistel (F), in Fluidverbindung gesetzt zu werden, und wobei die Blutbehandlungsvorrichtung zumindest eine Druckerzeugungseinrichtung (3, 8, 19, 24, 26) aufweist, die geeignet ist, auf den arteriellen (28) und/oder den venösen (41) Abschnitt einzuwirken, **dadurch gekennzeichnet, dass** die Blutbehandlungsvorrichtung eine Steuereinheit (30) zur Bestimmung eines Drucks in dem Blutgefäß (A, V, F) gemäß zumindest einem der Ansprüche 1 bis 12 aufweist.

## Claims

**1.** A control unit (30) for determining a pressure in a blood vessel (A, V, F), in particular in an arteriovenous fistula (F), which is in fluid connection with at least one section (28, 41) of a blood line system (39), in particular of an extracorporeal blood circulation (II), wherein at least one pressure-generating device (3, 8, 19, 24, 26) is assigned to the blood line system (39) and is suitable for acting on the section (28, 41), and wherein the control unit (30) is configured to carry out the following steps:

a) ensuring that none of the at least one pressure-generating device (3, 8, 19, 24, 26) acts on said section (28, 41),
b) interrupting the fluid connection of the section (28, 41) with the blood vessel (A, V, F) by triggering an interruption means (11, 29),
c) adjusting the pressure in said section (28, 41) at a predetermined target value, in particular at the ambient pressure, with the help of a pressure sensor (13, 14) in said section (28, 41),
d) restoring the fluid connection of said section (28, 41) with the blood vessel (A, V, F) by triggering the interruption means (11, 29), and
e) measuring the pressure established in said section (28, 41) with the help of the pressure sensor (13, 14).

**2.** The control unit (30) according to claim 1, which is configured to carry out the steps a) through e) on an arterial section (28) of a blood supply line (2) and/or on a venous section (41) of a blood return line (9) of the blood line system (39).

**3.** The control unit (30) according to claim 1 or 2, which is configured to carry out step a) by triggering the at least one pressure-generating device (3, 8, 19, 24, 26).

**4.** The control unit (30) according to at least one of the preceding claims, which is configured as a pressure-generating device (3, 8, 19, 24, 26) to trigger a blood pump (3), in particular a peristaltic pump (3).

**5.** The control unit (30) according to claim 4, which is configured to trigger the blood pump (3) in such a way that said pump runs in reverse and thereby reduces the vacuum in said section (28, 41) and then increases it to the target value, in particular the ambient pressure.

**6.** The control unit according to at least one of the preceding claims, which is configured as a pressure-generating device in step a) and/or c) to trigger a pump, in particular an ultrafiltration pump (8), in a dialysis fluid circulation (III), which is in fluid connection with the blood line system (39).

**7.** The control unit according to at least one of the preceding claims, which is configured to carry out steps a) through e) in a blood treatment device, such as a device for hemolysis, hemofiltration and/or hemodiafiltration.

**8.** The control unit according to at least one of the preceding claims, which is configured to carry out steps a) through e) at least once during a blood treatment session.

**9.** The control unit according to at least one of the preceding claims, which is configured to carry out steps a) through e) during a plurality of blood treatment sessions.

**10.** The control unit according to at least one of the preceding claims, which is configured to trigger at least one additional interruption means (42, K2) to interrupt the fluid connection between the at least one section (28, 41) and the remaining blood line system (39).

**11.** The control unit according to at least one of the preceding claims, which is configured to trigger an evaluation unit (34), so that the evaluation unit (34) subtracts the hydrostatic pressure applied to the pressure sensor (13, 14) from the pressure measured in said section (28, 41) in step e).

**12.** The control unit according to at least one of the preceding claims, which is configured to transmit the pressure in the blood vessel (A, V, F) and/or the pressure measured in step e) in said section (28, 41) to an evaluation unit (34), which is configured to evaluate a plurality of pressure values with regard to a developing stenosis.

**13.** A method for determining a pressure in a blood vessel (A, V, F), in particular in an arteriovenous fistula (F), which is in fluid connection with at least one section (28, 41) of a blood line system (39), in particular of an extracorporeal blood circulation (II), wherein at least one pressure-generating device (3, 8, 19, 24, 26), which is suitable for acting on said section (28, 41), is provided, wherein the method has the following steps:

> a) ensuring that none of the at least one pressure-generating device (3, 8, 19, 24, 26) acts on said section (28, 41),
> b) interrupting the fluid connection of the section (28, 41) with the blood vessel (A, V, F),
> c) adjusting the pressure in said section at a predetermined target value, in particular at the ambient pressure,
> d) restoring the fluid connection of said section (28, 41) with the blood vessel (A, V, F), and
> e) measuring the pressure established in the section (28, 41).

**14.** The method according to claim 13, **characterized in that** at least one of steps a) through e) is carried out by a control unit (30).

**15.** A blood treatment device for extracorporeal blood treatment, in particular for hemodialysis, hemofiltration and/or hemodiafiltration, provided for accommodating a blood line system (39) of an extra-corporeal blood circulation (II), wherein the blood line system (39) has an arterial section (28) of a blood supply line (2) and/or has a venous section (41) of a blood return line (9), wherein the arterial section (28) and the venous section (41) are provided for being put in fluid connection with a blood vessel (A, V, F), in particular an arterial venous fistula (F), and wherein the blood treatment device has at least one pressure-generating device (3, 8, 19, 24, 26) which is suitable for acting on the arterial section (28) and/or the venous section (41), **characterized in that** the blood treatment device has a control unit (30) for determining a pressure in the blood vessel (A, V, F) according to at least one of claims 1 through 12.

**Revendications**

**1.** Unité de commande (30) destinée à déterminer une pression dans un vaisseau sanguin (A, V, F), notamment dans une fistule (F) artério-veineuse, qui est en relation fluidique avec au moins un tronçon (28, 41) d'un système de conduits sanguins (39), notamment d'un système sanguin (II) extracorporel, au système de conduits sanguins (39) étant associé au moins un système générateur de pression (3, 8, 19, 24, 26) qui est apte à agir sur le tronçon (28, 41) et l'unité de commande (30) étant configurée pour réaliser les étapes suivantes, consistant à :

a) s'assurer qu'aucun des au moins un système générateur de pression (3, 8, 19, 24, 26) n'agisse sur le tronçon (28, 41),
b) interrompre la liaison fluidique du tronçon (28, 41) avec le vaisseau sanguin (A, V, F) par actionnement d'un moyen d'interruption (11, 29),
c) régler la pression dans le tronçon (28, 41) à une valeur de consigne prédéfinie, notamment à la pression environnante, à l'aide d'un capteur de pression (13, 14) dans le tronçon (28, 41),
d) rétablir la liaison fluidique du tronçon (28, 41) avec le vaisseau sanguin (A, V, F) par actionnement du moyen d'interruption (11, 29) et
e) mesurer une pression qui s'établit dans le tronçon (28, 41), à l'aide du capteur de pression (13, 14).

2. Unité de commande (30) selon la revendication 1, qui est configurée pour réaliser les étapes a) à e) sur un tronçon artériel (28) d'un conduit d'afflux sanguin (2) et/ou sur un tronçon veineux (41) d'un conduit de reflux sanguin (9) du système de conduits sanguins (39).

3. Unité de commande (30) selon la revendication 1 ou la revendication 2, qui est configurée pour réaliser l'étape a) en ce que l'au moins un système générateur de pression (3, 8, 19, 24, 26) est actionné.

4. Unité de commande (30) selon au moins l'une quelconque des revendications précédentes, qui est configurée pour actionner, en tant que système générateur de pression (3, 8, 19, 24, 26) une pompe à sang (3), notamment une pompe péristaltique (3).

5. Unité de commande (30) selon la revendication 4, qui est configurée pour actionner la pompe à sang (3), de sorte qu'elle fonctionne en sens inverse et réduise de ce fait une dépression dans le tronçon (28, 41) et la relève à une valeur de consigne, notamment la pression environnante.

6. Unité de commande selon au moins l'une quelconque des revendications précédentes, qui est configurée pour actionner dans l'étape a) et/ou c) en tant que système générateur de pression une pompe, notamment une pompe d'ultrafiltration (8) dans un circuit de liquide de dialyse (III) qui est en liaison fluidique avec le système de conduits sanguins (39).

7. Unité de commande selon au moins l'une quelconque des revendications précédentes, qui est configurée pour réaliser les étapes a) à e) dans un dispositif de traitement sanguin, comme par exemple un dispositif d'hémodialyse, d'hémofiltration et/ou d'hémodiafiltration.

8. Unité de commande selon au moins l'une quelconque des revendications précédentes, qui est configurée pour réaliser les étapes a) à e) au moins une fois pendant une session de traitement sanguin.

9. Unité de commande selon au moins l'une quelconque des revendications précédentes, qui est configurée pour réaliser les étapes a) à e) pendant une pluralité de sessions de traitement sanguin.

10. Unité de commande selon au moins l'une quelconque des revendications précédentes, qui est configurée pour actionner au moins un moyen d'interruption (42, K2) supplémentaire pour interrompre la liaison fluidique entre l'au moins un tronçon (28, 41) et le reste du système de conduits sanguins (39).

11. Unité de commande selon au moins l'une quelconque des revendications précédentes, qui est configurée pour actionner une unité d'évaluation (34), pour que l'unité d'évaluation (34) ôte une pression hydrostatique appliquée sur le capteur de pression (13, 14) de la pression mesurée pendant l'étape e) dans le tronçon (28, 41).

12. Unité de commande selon au moins l'une quelconque des revendications précédentes, qui est configurée pour transmettre la pression dans le vaisseau sanguin (A, V, F) et/ou la pression mesurée pendant l'étape e) dans le tronçon (28, 41) à une unité d'évaluation (34) qui est configurée pour évaluer une pluralité de valeurs de pression en ce qui concerne la sténose qui est en train de se former.

13. Procédé destiné à déterminer une pression dans un vaisseau sanguin (A, V, F), notamment dans une fistule (F) artério-veineuse qui est en liaison fluidique avec au moins un tronçon (28, 41) d'un système de conduits sanguins (39), notamment d'un système sanguin (II) extracorporel, au moins un système générateur de pression (3, 8, 19, 24, 26) étant prévu qui est apte à agir sur le tronçon (28, 41), le procédé comportant les étapes suivantes, consistant à :

a) s'assurer qu'aucun des au moins un système générateur de pression (3, 8, 19, 24, 26) n'agisse sur le tronçon (28, 41),
b) interrompre la liaison fluidique du tronçon (28, 41) avec le vaisseau sanguin (A, V, F),
c) régler la pression à une valeur de consigne notamment à une pression environnante,
d) rétablir la liaison fluidique du tronçon (28, 41) avec le vaisseau sanguin (A, V, F) et

e) mesurer une pression qui s'établit dans le tronçon (28, 41).

**14.** Procédé selon la revendication 13, **caractérisé en ce qu'**au moins l'une des étapes a) à e) est réalisée par une unité de commande (30).

**15.** Dispositif de traitement sanguin pour le traitement sanguin extracorporel, notamment pour l'hémodialyse, l'hémofiltration et/ou l'hémodiafiltration, prévu pour recevoir un système de conduits sanguins (39) d'un système sanguin (II) extracorporel, le système de conduits sanguins (39) possédant un tronçon artériel (28) d'un conduit d'afflux sanguin (2) et/ou un tronçon veineux (41) d'un conduit de reflux sanguin (9), le tronçon artériel (28) et le tronçon veineux (41) étant prévus pour être mis en liaison fluidique avec un vaisseau sanguin (A, V, F), notamment une fistule (F) artério-veineuse et le dispositif de traitement sanguin comportant au moins un système générateur de pression (3, 8, 19, 24, 26) qui est apte à agir sur le tronçon artériel (28) et/ou veineux (41), **caractérisé en ce que** le dispositif de traitement sanguin comporte une unité de commande (30) destinée à déterminer une pression dans le vaisseau sanguin (A, V, F) selon au moins l'une quelconque des revendications 1 à 12.

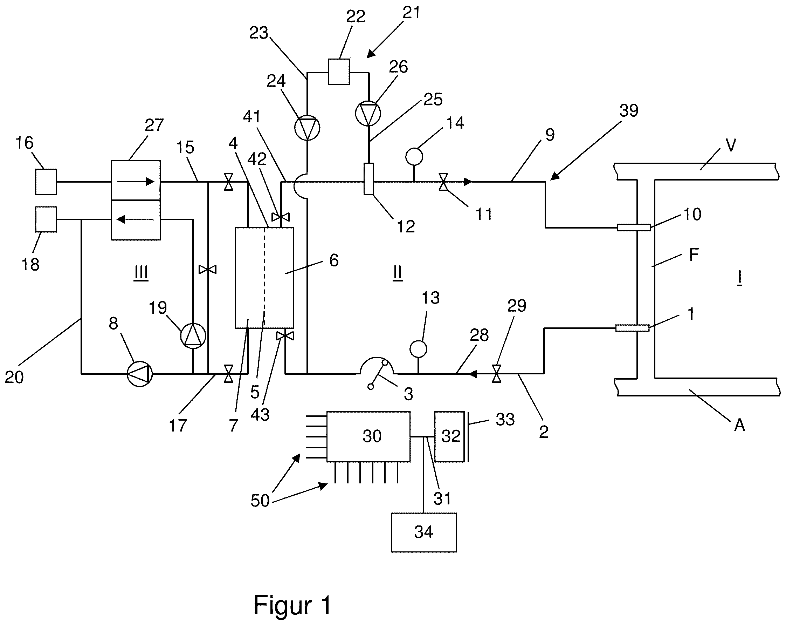

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19917197 C1
- DE 102008061122 A1
- DE 102008059379 A1